(19) Europäisches Patentamt
European Patent Office
Office européen des brevets

(11) EP 1 258 481 B1

(12) **EUROPÄISCHE PATENTSCHRIFT**

(45) Veröffentlichungstag und Bekanntmachung des Hinweises auf die Patenterteilung:
**18.06.2008 Patentblatt 2008/25**

(51) Int Cl.:
***C07D 251/70*** *(2006.01)* ***A61K 8/49*** *(2006.01)*

(21) Anmeldenummer: **02007892.9**

(22) Anmeldetag: **09.04.2002**

(54) **Enaminotriazine als UV-Filter**

Enaminotriazines as UV filters

Enaminotriazine en tant que filtres UV

(84) Benannte Vertragsstaaten:
**AT BE CH CY DE DK ES FI FR GB GR IE IT LI LU MC NL PT SE TR**
Benannte Erstreckungsstaaten:
**RO SI**

(30) Priorität: **18.05.2001 DE 10124332**

(43) Veröffentlichungstag der Anmeldung:
**20.11.2002 Patentblatt 2002/47**

(73) Patentinhaber: **BASF SE**
**67056 Ludwigshafen (DE)**

(72) Erfinder:
- **Heidenfelder, Thomas, Dr.**
**67125 Dannstadt (DE)**
- **Tiefensee, Kristin, Dr.**
**67098 Bad Duerkheim (DE)**
- **Wünsch, Thomas, Dr.**
**67346 Speyer (DE)**

(56) Entgegenhaltungen:
**EP-A- 0 507 691**
**EP-A- 0 790 243**
**EP-A- 0 852 137**
**EP-A- 0 895 776**

- **HASHIDA, YOJI ET AL: "Preparation and reactions of isocyano-1,3,5-triazines" J. HETEROCYCL. CHEM. (1989), 26(4), 901-5 , XP002210051**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft die Verwendung von substituierten Enaminotriazinen als UV-Filter allein oder zusammen mit weiteren UV-Filtern in kosmetischen und pharmazeutischen Zubereitungen und neue Enaminotriazine.

Die in kosmetischen und pharmazeutischen Zubereitungen eingesetzten Lichtschutzmittel haben die Aufgabe, schädigende Einflüsse des Sonnenlichts auf die menschliche Haut zu verhindern oder zumindest in ihren Auswirkungen zu reduzieren. Daneben dienen diese Lichtschutzmittel aber auch dem Schutz weiterer Inhaltsstoffe vor Zerstörung oder Abbau durch UV-Strahlung. In haarkosmetischen Formulierungen soll eine Schädigung der Keratinfaser durch UV-Strahlen vermindert werden.

Das an die Erdoberfläche gelangende Sonnenlicht hat einen Anteil an UV-B- (280 bis 320 nm) und an UV-A-Strahlung (> 320 nm), welche sich direkt an den Bereich des sichtbaren Lichtes anschließen. Der Einfluß auf die menschliche Haut macht sich besonders bei der UV-B-Strahlung durch Sonnenbrand bemerkbar. Dementsprechend bietet die Industrie eine größere Zahl von Substanzen an, welche die UV-B-Strahlung absorbieren und damit den Sonnenbrand verhindern.

Nun haben dermatologische Untersuchungen gezeigt, daß auch die UV-A-Strahlung durchaus Hautschädigungen und Allergien hervorrufen kann, indem beispielsweise das Keratin oder Elastin geschädigt wird. Hierdurch werden Elastizität und Wasserspeichervermögen der Haut reduziert, d.h. die Haut wird weniger geschmeidig und neigt zur Faltenbildung. Die auffallend hohe Hautkrebsläufigkeit in Gegenden starker Sonneneinstrahlung zeigt, daß offenbar auch Schädigungen der Erbinformationen in den Zellen durch Sonnenlicht, speziell durch UV-A-Strahlung, hervorgerufen werden. All diese Erkenntnisse lassen daher die Entwicklung effizienter Filtersubstanzen für den UV-A- und UV-B-Bereich notwendig erscheinen.

Es besteht ein wachsender Bedarf an Lichtschutzmitteln für kosmetische und pharmazeutische Zubereitungen, deren Absorptionsmaxima im Bereich von ca. 280 bis 380 nm liegen sollten. Um mit einer möglichst geringen Einsatzmenge die gewünschte Wirkung zu erzielen, sollten derartige Lichtschutzmittel zusätzlich eine hoch spezifische Extinktion aufweisen. Außerdem müssen Lichtschutzmittel für kosmetische Präparate noch eine Vielzahl weiterer Anforderungen erfüllen, beispielsweise gute Formulierbarkeit in kosmetischen Präparaten, hohe Stabilität der mit ihnen hergestellten Emulsionen, toxikologische Unbedenkbarkeit, sowie geringen Eigengeruch und geringe Eigenfärbung.

Obgleich bereits eine Vielzahl von UV-Lichtschutzmitteln bekannt ist, ist die Kombination der Eigenschaften aus hoher Extinktion im UV-A- und/oder UV-B-Bereich und Photostabilität nur unzureichend verwirklicht. Es besteht daher ein Bedarf an UV-Filtern, die diese Erfordernisse in besonders hohem Maße verwirklichen.

Die Aufgabe, neue Lichtschutzmittel, die diesen Erfordernissen genügen, vorzuschlagen, wurde erfindungsgemäß gelöst mit der Verwendung von Enaminotriazinen der Formel I

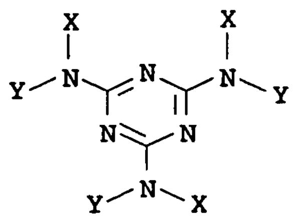

(I),

in der die Reste Y unabhängig voneinander Wasserstoff, einen gegebenenfalls substituierten aliphatischen, cycloaliphatischen, araliphatischen oder aromatischen Rest mit jeweils bis zu 18 C-Atomen bedeuten, die Reste X gleich oder verschieden sind, wobei mindestens einer der Reste X den Ethenylrest der Formel II

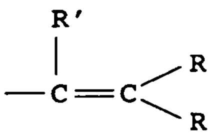

(II)

bedeutet und X ferner Wasserstoff bedeuten kann und wobei in der Formel II die Reste Reste R gleich oder verschieden sind und Cyan, gegebenenfalls verestertes Carboxy, substituiertes Carbonyl, gegebenenfalls substituiertes Aminocarbonyl, gegebenenfalls verestertes Sulfo, substituiertes Sulfonyl oder gegebenenfalls verestertes Phosphono (das ist der Rest $(HO)_2OP$-) bedeuten und wobei zwei benachbarte Reste R gegebenenfalls einen Ring bilden können und die Reste R' gleich oder verschieden sind und Wasserstoff, einen gegebenenfalls substituierten aliphatischen, cycloaliphatischen oder aromatischen Rest mit jeweils bis 18 C-Atomen bedeuten, als UV-Filter zur Herstellung von kosmetischen oder pharmazeutischen Zubereitungen zum Schutz der menschlichen Haut oder menschlichen Haare gegen Sonnenstrahlen, allein oder zusammen mit an sich für kosmetische Zubereitungen bekannten, im UV-Bereich absorbierenden Verbindungen.

Bevorzugt ist dabei die Verwendung von Verbindungen der Formel I, in der Y Wasserstoff und einer oder mehrere der Reste X einen Ethenylrest der Formel IIa

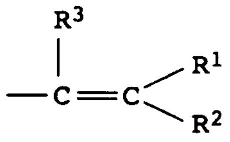

(IIa)

bedeuten, in der die Reste $R^1$ und $R^2$ gleich oder verschieden sind und $-COOR^4$, $-COR^4$, $-CON(R^4)_2$, -CN, $-SO_2R^4$, $-SO_2OR^4$ oder $-PO(OR^4)_2$ bedeuten, wobei die Reste $R^4$ unabhängig voneinander Wasserstoff einen aliphatischen, cycloaliphatischen, araliphatischen oder aromatischen Rest, die jeweils gegebenenfalls substituiert sein können, bedeuten und wobei benachbarte Reste $R^1$ und $R^2$ gegebenenfalls einen Ring bilden können und $R^3$ für Wasserstoff oder einen aliphatischen, cycloaliphatischen, araliphatischen oder aromatischen Rest, die jeweils substituiert sein können, steht.

Besonders bevorzugt sind Verbindungen der Formel I, bei denen $R^3$ Wasserstoff, $R^1$ und $R^2$ gleich oder verschieden sind und $-COOR^5$, $-COR^5$ oder -CN bedeuten, wobei $R^5$ für einen aliphatischen oder gegebenenfalls substituierten aromatischen Rest mit bis zu 18 C-Atomen steht.

Die Erfindung betrifft gleichermaßen Lichtschutzmittel enthaltende kosmetische oder pharmazeutische Zubereitungen in Form von Ölen, Öl-in-Wasser- und Wasser-in-Öl-Emulsionen, Cremes, Pasten, Lippenschutzstiftmassen oder fettfreien Gelen zum Schutz der menschlichen Haut oder menschlichen Haare gegen Sonnenstrahlen, allein oder zusammen mit an sich für kosmetische Zubereitungen bekannten, im UV-Bereich absorbierenden Verbindungen, die als wesentliches UV-Filter wirksame Mengen von Enaminotriazinen der Formel I

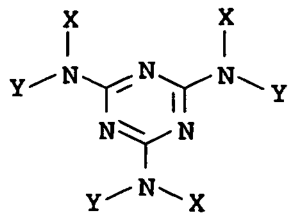

(I),

enthalten, in der die Reste Y unabhängig voneinander Wasserstoff, einen gegebenenfalls substituierten aliphatischen, cycloaliphatischen, araliphatischen oder aromatischen Rest mit jeweils bis zu 18 C-Atomen bedeuten, die Reste X gleich oder verschieden sind, wobei mindestens einer der Reste X den Ethenylrest der Formel II

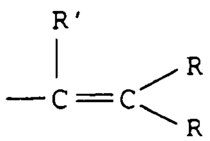

(II)

bedeutet und X ferner Wasserstoff bedeuten kann und wobei in der Formel II die Reste Reste R gleich oder verschieden sind und Cyan, gegebenenfalls verestertes Carboxy, substituiertes Carbonyl, gegebenenfalls substituiertes Aminocarbonyl, gegebenenfalls verestertes Sulfo, substituiertes Sulfonyl oder gegebenenfalls verestertes Phosphono bedeuten und wobei zwei benachbarte Reste R gegebenenfalls einen Ring bilden können, und die Reste R' gleich oder verschieden sind und Wasserstoff, einen gegebenenfalls substituierten aliphatischen, cycloaliphatischen oder aromatischen Rest mit jeweils bis 18 C-Atomen bedeuten.

Schließlich betrifft die Erfindung auch als neue Verbindungen Tris-(ethenylamino)-triazine der Formel III

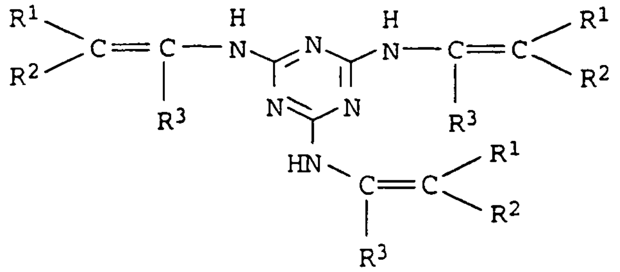

(III),

in der die Reste $R^1$ und $R^2$ gleich oder verschieden sind und $-COOR^4$, $-COR^4$, $-CON(R^4)_2$, -CN, $-SO_2R^4$, $-SO_2OR^4$ oder $-PO(OR^4)_2$ bedeuten, wobei die Reste $R^4$ unabhängig voneinander Wasserstoff einen aliphatischen, cycloaliphatischen, araliphatischen oder aromatischen Rest, die jeweils gegebenenfalls substituiert sein können, bedeuten und wobei zwei benachbarte Reste R gegebenenfalls einen Ring bilden können und $R^3$ für Wasserstoff oder einen aliphatischen, cycloaliphatischen, araliphatischen oder aromatischen Rest, die jeweils substituiert sein können, steht.

In allen vorstehend genannten Verbindungen kann die Enamingruppe in der E- oder Z-Konfiguration vorliegen.

Enaminotriazine sind aus mehreren Literaturstellen, z.B. aus Hashida, Y; Imai, A.; Sekiguchi, S; J. Heterocyclic Chem. 1989, 26, 901-5, WO 99/02495 und WO 9847893 bekannt.

Ferner sind von Kreutzberger, A; Grundmann, C; J. Org. Chem. 1961, 4, 1121-1126 schon Enamino(hexahydro) triazine beschrieben worden. In keiner dieser Literaturstellen ist die Verwendung dieser Verbindungen als Lichtschutzmittel in kosmetischen Zubereitungen offenbart oder nagegelegt.

Schließlich sind Enaminosubstituenten tragende Aromaten als Lichtschutzmittel in kosmetischen Zubereitungen aus EP-A 895 776 und EP-A 852 137 bekannt. In letzterer Schrift ist auch die allgemeine Angabe zu finden, daß der aromatische Rest auch ein Heteroaromat sein kann.

Aus diesen Literaturstellen war jedoch nicht abzuleiten, daß sich die erfindungsgemäß zu verwendenden Enaminotriazine durch ihre besonders vorteilhaften Eigenschaften, wie sehr hohe Extinktion und hohe Photostabilität auszeichnen.

Besonders bevorzugt ist die Verwendung von Verbindungen der Formel I, in der $R^3$ für Wasserstoff und $R^1$ und $R^2$ unabhängig voneinander für CN, $COOR^4$ und $COR^4$ stehen, wobei $R^4$ gleiche oder verschiedene offenkettige oder verzweigte aliphatische oder gegebenenfalls substituierte, aromatische Reste mit bis zu 8 C-Atomen bedeuten.

Als Substituenten kommen sowohl lipophile als auch hydrophile Substituenten mit z.B. bis zu 20 C-Atomen in Betracht. Lipophile d.h. die Öllöslichkeit der Verbindungen der Formel I verstärkende Reste sind z.B. aliphatische oder cycloaliphatische Reste, insbesondere Alkylreste mit 1 bis- 18 C-Atomen, Alkoxy-, Mono- und Dialkylamino-, Alkoxycarbonyl-, Mono- und Dialkylaminocarbonyl-, Mono- und Dialkylaminosulfonylreste, ferner Cyan-, Nitro-, Brom-, Chlor-, Iod- oder Fluorsubstituenten.

Hydrophile d.h. die Wasserlöslichkeit der Verbindungen der Formel I ermöglichende Reste sind z.B. Carboxy- und Sulfoxyreste und insbesondere deren Salze mit beliebigen physiologisch verträglichen Kationen, wie die Alkalisalze oder wie die Trialkylammoniumsalze, wie Tri-(hydroxyalkyl)-ammoniumsalze oder die 2-Methylpropan-1-ol-2-ammoniumsalze. Ferner kommen Alkylammoniumreste mit beliebigen physiologisch verträglichen Anionen in Betracht. Schlechtlösliche Verbindungen der Formel I können in mikronisierter Form in kosmetischen Zubereitungen eingesetzt werden.

Als Alkoxyreste kommen solche mit 1 bis 12 C-Atomen, vorzugsweise mit 1 bis 8 C-Atomen in Betracht.

Beispielsweise sind zu nennen:

| | |
|---|---|
| methoxy | isopropoxy- |
| n-propoxy- | 1-methylpropoxy- |
| n-butoxy- | n-pentoxy- |
| 2-methylpropoxy- | 3-methylbutoxy- |
| 1,1-dimethylpropoxy- | 2,2-dimethylpropoxy- |
| hexoxy- | 1-methyl-1-ethylpropoxy- |
| heptoxy- | octoxy- |
| 2-ethylhexoxy- | |

Als Mono- oder Dialkylaminoreste kommen z.B solche in Betracht, die Alkylreste mit 1 bis 8 C-Atomen enthalten, wie Methyl-, n-Propyl-, n-Butyl-, 2-Methylpropyl-, 1,1-Dimethylpropyl-, Hexyl-, Heptyl-, 2-Ethylhexyl-, Isopropyl-, 1-Methylpropyl-, n-Pentyl-, 3-Methylbutyl-, 2,2-Dimethylpropyl-, 1-Methyl-1-ethylpropyl- und Octyl in Betracht. Diese Reste sind gleichermaßen in den Mono- und Dialkylaminocarbonyl- und Sulfonylresten enthalten.

Die erfindungsgemäß zu verwendenden Verbindungen der Formel I und III können nach der Gleichung durch Umsetzung einer Verbindung mit aktivierter Methylengruppe mit Melamin in Gegenwart von Orthoformiat gemäß dem folgenden Schema

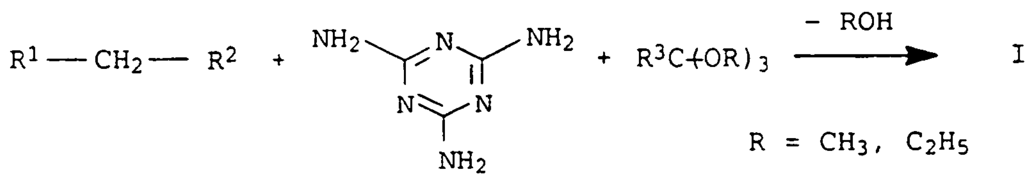


durch Kondensation in an sich bekannter Weise hergestellt werden, wobei $R^1$ bis $R^3$ die oben genannte Bedeutung haben.

Als aktive Methylengruppen enthaltende Verbindungen $R^1$-$CH_2$-$R^2$ kommen z.B. Cyanessigsäureester, Cyanessigsäureamide, Cyanessigsäure, Malonester, Malonsäureamide, Malonsäure, Acetessigester, Acetessigsäureamide, Acetessigsäure, 1,3-Diketone, Malodinitril und $\alpha$-Cyanoketone in Betracht.

Je nachdem wie das Verhältnis der Methylenverbindung (und Orthoformiat) zu Melamin gewählt wird, erhält man Mono-, Di- oder Trisubstitution des Melamins. Bevorzugt ist Umsetzung mit der mindestens 3-fach molaren Menge, so daß vorherrschend die trisubstituierte Verbindung erhalten wird. Einzelheiten der Synthesemethode sind den Angaben der EP-A 0 852 137 zu entnehmen.

Die Lichtschutzmittel enthaltenden kosmetischen und pharmazeutischen Zubereitungen sind in der Regel auf der Basis eines Trägers hergestellt, der mindestens eine Ölphase enthält. Es sind aber auch Zubereitungen allein auf wäßriger Basis bei Verwendung von Verbindungen mit hydrophilen Substituenten möglich. Demgemäß kommen Öle, Öl-in-Wasser- und Wasser-in-Öl-Emulsionen, Cremes und Pasten, Lippenschutzstiftmassen oder fettfreie Gele in Betracht.

Solche Sonnenschutzpräparate können demgemäß in flüssiger, pastöser oder fester Form vorliegen, beispielsweise als Wasser-in-Öl-Cremes, Öl-in-Wasser-Cremes und -Lotionen, Aerosol-Schaumcremes, Gele, Öle, Fettstifte, Puder, Sprays oder alkoholischwäßrige Lotionen.

Übliche Ölkomponenten in der Kosmetik sind beispielsweise Paraffinöl, Glycerylstearat, Isopropylmyristat, Diisopropyladipat, 2-Ethylhexansäurecetylstearylester, hydriertes Polyisobuten, Vaseline, Caprylsäure/Caprinsäure-Triglyceride, mikrokristallines Wachs, Lanolin und Stearinsäure.

Übliche kosmetische Hilfsstoffe, die als Zusätze in Betracht kommen können, sind z.B. Co-Emulgatoren, Fette und Wachse, Stabilisatoren, Verdickungsmittel, biogene Wirkstoffe, Filmbildner, Duftstoffe, Farbstoffe, Perlglanzmittel, Konservierungsmittel, Pigmente, Elektrolyte (z.B. Magnesiumsulfat) und pH-Regulatoren. Als Co-Emulgatoren kommen vorzugsweise bekannte W/O- und daneben auch O/W-Emulgatoren wie etwa Polyglycerinester, Sorbitanester oder teilveresterte Glyceride in Betracht. Typische Beispiele für Fette sind Glyceride; als Wachse sind u.a. Bienenwachs, Paraffinwachs oder Mikrowachse gegebenenfalls in Kombination mit hydrophilen Wachsen zu nennen. Als Stabilisatoren können Metallsalze von Fettsäuren wie z.B. Magnesium-, Aluminium- und/oder Zinkstearat eingesetzt werden. Geeignete Verdickungsmittel sind beispielsweise vernetzte Polyacrylsäuren und deren Derivate, Polysaccharide, insbesondere Xanthan-Gum, Guar-Guar, Agar-Agar, Alginate und Tylosen, Carboxymethylcellulose und Hydroxyethylcellulose, ferner Fettalkohole, Monoglyceride und Fettsäuren, Polycrylate, Polyvinylalkohol und Polyvinylpyrrolidon. Unter biogenen Wirkstoffen sind beispielsweise Pflanzenextrakte, Eiweißhydrolysate und Vitaminkomplexe zu verstehen. Gebräuchliche Filmbildner sind beispielsweise Hydrocolloide wie Chitosan, mikrokristallines Chitosan oder quaterniertes Chitosan, Polyvinylpyrrolidon, Vinylpyrrolidon-Vinylacetat-Copolymerisate, Polymere der Acrylsäurereihe, quaternäre Cellulose-Derivate und ähnliche Verbindungen. Als Konservierungsmittel eignen sich beispielsweise Formaldehydlösung, p-Hydroxybenzoat oder Sorbinsäure. Als Perlglanzmittel kommen beispielsweise Glycoldistearinsäureester wie Ethylenglycoldistearat, aber auch Fettsäuren und Fettsäuremonoglycolester in Betracht. Als Farbstoffe können die für kosmetische Zwecke geeigneten und zugelassenen Substanzen verwendet werden, wie sie beispielsweise in der Publikation "Kosmetische Färbemittel" der Farbstoffkoimmission der Deutschen Forschungsgemeinschaft, veröffentlicht im Verlag Chemie, Weinheim, 1984, zusammengestellt sind. Diese Farbstoffe werden üblicherweise in Konzentration von 0,001 bis 0,1 Gew.-%, bezogen auf die gesamte Mischung, eingesetzt.

Der Gesamtanteil der Hilfs- und Zusatzstoffe kann 1 bis 80, vorzugsweise 6 bis 40 Gew.-% und der nicht wäßrige Anteil ("Aktivsubstanz") 20 bis 80, vorzugsweise 30 bis 70 Gew.-% - bezogen auf die Mittel - betragen. Die Herstellung der Mittel kann in an sich bekannter Weise, d.h. beispielsweise durch Heiß-, Kalt-, Heiß-Heiß/Kalt- bzw. PIT-Emulgierung erfolgen. Hierbei handelt es sich um ein rein mechanisches Verfahren, eine chemische Reaktion findet nicht statt.

Schließlich können weitere an sich bekannte im UV-A-Bereich absorbierenden Substanzen mitverwendet werden, sofern sie im Gesamtsystem der erfindungsgemäß zu verwendenden Kombination aus UV-B und UV-A Filter stabil sind.

Gegenstand der vorliegenden Erfindung sind weiterhin kosmetische und pharmazeutische Zubereitungen, die 0,1 bis 10 Gew.-%, vorzugsweise 1 bis 7 Gew.-%, bezogen auf die gesamte Menge der kosmetischen und pharmazeutischen Zubereitung, eine oder mehrere der Verbindungen der Formel I zusammen mit an sich für kosmetische und pharmazeutische Zubereitungen bekannten, im UV-B-Bereich absorbierenden Verbindungen als Lichtschutzmittel enthalten, wobei die Verbindungen der Formel I in der Regel in geringerer Menge als die UV-B-absorbierenden Verbindungen eingesetzt werden.

Als UV-B-Filtersubstanzen, die in Kombination mit den erfindungsgemäß zu verwendenden Verbindungen der Formel I angewandt werden, kommen beliebige UV-B-Filtersubstanzen in Betracht. Beispielsweise sind zu nennen:

| Nr. | Stoff | CAS-Nr. (=Säure) |
|---|---|---|
| 1 | 4-Aminobenzoesäure | 150-13-0 |
| 2 | 3-(4'Trimethylammonium)-benzylidenbornan-2-on-methylsulfat | 52793-97-2 |
| 3 | 3,3,5-Trimethyl-cyclohexyl-salicylat (Homosalatum) | 118-56-9 |
| 4 | 2-Hydroxy-4-methoxy-benzophenon (Oxybenzonum) | 131-57-7 |
| 5 | 2-Phenylbenzimidazol-5-sulfonsäure und ihre Kalium-, Natrium- u. Triethanolaminsalze | 27503-81-7 |
| 6 | 3,3'-(1,4-Phenylendimethin)-bis(7,7-dimethyl-2-oxobicyclo[2.2.1]heptan-1-methansulfonsäure) und ihre Salze | 90457-82-2 |
| 7 | 4-Bis(polyethoxy)amino-benzoesäurepolyethoxyethylester | 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-2-isoamylester | 7/6/7-10-2 |
| 11 | 4-Methoxy-zimtsäure-2-ethylhexylester | 5466-77-3 |
| 12 | 2-Hydroxy-4-methoxy-benzophenon-5-sulfon-(Sulisobenzonum) und das Natriumsalz | 4065-45-6 |
| 13 | 3-(4'-Sulfo)benzyliden-bornan-2-on und Salze | 58030-58-6 |
| 14 | 3-(4'-Methyl)benzyliden-bornan-2-on | 36861-47-9 |
| 15 | 3-Benzylidenbornan-2-on | 16087-24-8 |
| 16 | 1-(4'-Isopropylphenyl)-3-phenylpropan-1,3-dion | 63260-25-9 |
| 17 | 4-Isopropylbenzylsalicylat | 94134-93-7 |
| 18 | 2,4,6-Trianilin-(o-carbo-2'-ethylhexyl-1'-oxy)-1,3,5-triazin | 88122-99-0 |
| 19 | 3-Imidazol-4-yl-acrylsäure und ihr Ethylester | 104-98-3* |
| 20 | 2-Cyano-3,3-diphenylacrylsäureethylester | 5232-99-5 |
| 21 | 2-Cyano-3,3-diphenylacrylsäure-2'-ethylhexylester | 6197-30-4 |
| 22 | Menthyl-o-aminobenzoate oder: 5-Methyl-2-(1-methylethyl)-2-aminobenzoate | 134-09-8 |
| 23 | Glyceryl p-aminobenzoat oder: 4-Aminobenzoesäure-1-glyceryl-ester | 136-44-7 |
| 24 | 2,2'-Dihydroxy-4-methoxybenzophenon (Dioxybenzone) | 131-53-3 |
| 25 | 2-Hydroxy-4-methoxy-4-methylbenzophenon (Mexonon) | 1641-17-4 |
| 26 | Triethanolamin Salicylat | 2174-16-5 |
| 27 | Dimethoxyphenylglyoxalsäure oder: 3,4-dimethoxy-phenyl-glyoxal-saures Natrium | |
| 28 | 3-(4'Sulfo)benzyliden-bornan-2-on und seine Salze | 56039-58-8 |

Falls die erfindungsgemäß zu verwendende Verbindung der Formel I dagegen einen UV-B-Filter darstellt, kann

sie selbstverständlich auch mit an sich bekannten UV-A-Filtern oder auch Breitbandabsorbern kombiniert werden.

Solche UV-A- und Breitbandabsorber sind z.B.

| Nr. | Stoff | CAS-Nr. (=Säure) |
|---|---|---|
| 29 | 1,1-Bis(neopentyloxycarbonyl)-4,4-diphenyl-1,3-Butadien | gem. EPA 916335 |
| 30 | 2-(4'-Diethylamino-2'-hydroxybenzoyl)benzoesäure-n-hexylester | 302776-68-7 |
| 31 | 2,2'-Methylen-bis-[6-(2H-benzotriazol-2-yl)-4-(1,1,3,3-tetramethylbutyl)-phenol (Tinosorb® M) | 103597-45-1 |
| 32 | 2,2'-[6-methoxyphenyl)-1,3,5-triazin-2,4-diyl]-bis[5-[(2-ethylhexyl)oxy]-phenol (Tinosorb® S) | 187393-00-6 |
| 33 | 2-[5,6-Disulfo(1H-benzimidazol-2-yl)phenyl]-1H-benzimidazol-5,6-disulfonsäure | 180898-37-7 |
| 34 | 2-(2H-1,2,3-Benzotriazol-2-yl)-4-methyl-6-(2-methyl-3-[1,3,3,3-tetramethyl-1-(trimethylsilyl)oxy]disiloxanyl]propyl)phenol | 155633-54-8 |

Schließlich sind auch mikronisierte Pigmente wie Titandioxid und Zinkoxid zu nennen.

Zum Schutz menschlicher Haare vor UV-Strahlen können die erfindungsgemäßen Lichtschutzmittel der Formel I in Shampoos, Lotionen, Gelen oder Emulsionen in Konzentrationen von 0,1 bis 10 Gew.-%, bevorzugt 1 bis 7 Gew.-% eingearbeitet werden. Die jeweiligen Formulierungen können dabei u.a. zum Waschen, Färben sowie zum Frisieren der Haare verwendet werden.

Die erfindungsgemäß zu verwendenden Verbindungen zeichnen sich in der Regel durch ein besonders hohes Absorptionsvermögen aus. Weiterhin sind sie gut in kosmetischen Ölen löslich und lassen sich leicht in kosmetische Formulierungen einarbeiten. Die mit den Verbindungen I hergestellten Emulsionen zeichnen sich besonders durch ihre hohe Stabilität, die Verbindungen I selber durch ihre hohe Photostabilität aus, und die mit I hergestellten Zubereitungen durch ihr angenehmes Hautgefühl aus.

Die erfindungsgemäße pharmazeutische Verwendung betrifft im wesentlichen die topische Anwendung. Sie erfolgt beispielsweise als Salbe, Creme, Gel, Spray, Lösung oder Lotion.

Beispiele:

I. Herstellung

Beispiel 1

0,1 mol Melamin 0,3 mol Cyanessigsäureethylester und 0,3 mol Triethylorthoformiat wurden 11 h auf 120°C erhitzt, wobei Ethanol abdestillierte. Nach Abkühlung wurden 200 ml Ethanol zugegeben und der ausgefallene Niederschlag abfiltriert. Der Rückstand wurde mit Petrolether gewaschen und bei 50°C im Vakuum getrocknet. Man erhielt 29,1 g (59 %) der Verbindung der Formel III, in der $R^3$ Wasserstoff, $R^1$ Cyan und $R^2$ Ethyloxycarbonyl bedeutet ($\lambda_{max}$ = 318 nm; $E_1^1 = 1784$)

In analoger Weise werden hoch wirksame Verbindungen der Formel I erhalten, wenn man als aktivierte Methylenverbindungen die folgenden Verbindungen als Ausgangsverbindungen verwendet:

Acetessigsäuremethylester
Acetessigsäureethylester
Acetessigsäure(2-ethylhexyl)ester
Acetylaceton
Dibenzoylmethan
Pivaloylacetonitril
Malonsäuredimethylester
Malonsäurediethylester
Malonsäuredi(2-ethylhexyl)ester
Benzoylacetonitril
Benzooxazol-2-yl-acetonitril

Benzoxazol-2-yl-essigsäure
Benzoxazol-2-yl-essigsäuremethylester
Benzoxazol-2-yl-essigsäureethylester
Benzoxazol-2-yl-essigsäure(2-ethylhexyl)ester
Cyanessigsäuremethylester
Cyanessigsäure(2-ethylhexyl)ester
l-Phenyl-1,3-butandion
Benzoylacetonitril
Malodinitril
Indan-1,3-dion
2,2-Dimethyl-1,3-dioxan-4,6-dion (Meldrumsäure)
Sulfonyldiacetonitril
2-Benzimidazoylacetonitril
(1H-Benzoimidazol-2-yl)-essigsäure
(1H-Benzoimidazol-2-yl)-essigsäuremethylester
(1H-Benzoimidazol-2-yl)-essigsäureethylester
(1H-Benzoimidazol-2-yl)-essigsäure(2-ethylhexyl)ester

Allgemeine Herstellvorschrift zur Herstellung von Emulsionen für kosmetische Zwecke

Alle öllöslichen Bestandteile werden in einem Rührkessel auf 85°C erwärmt. Wenn alle Bestandteile geschmolzen sind, bzw. als Flüssigphase vorliegen, wird die Wasserphase unter Homogenisieren eingearbeitet. Unter Rühren wird die Emulsion auf ca. 40°C abgekühlt, parfümiert, homogenisiert und dann unter ständigem Rühren auf 25°C abgekühlt.

Zubereitungen

Beispiel 2

| Zusammensetzung für die Lippenpflege | |
|---|---|
| ad 100 | Eucerinum anhydricum |
| 10,00 | Glycerin |
| 10,00 | Titanium Dioxid |
| 1,00 | Verbindung des Beispiels 1 |
| 8,00 | Octyl Methoxycinnamat |
| 5,00 | Zink Oxid |
| 4,00 | Castoröl |
| 4,00 | Pentaerythrithyl Stearat/Caprat/Caprylat/Adipat |
| 3,00 | Glyceryl Stearat SE |
| 2,00 | Bienenwachs |
| 2,00 | Microkristallines Wachs |
| 2,00 | Quaternium-18 Bentonit |
| 1,50 | PEG-45/Dodecyl Glycol Copolymer |

Beispiel 3

| Zusammensetzung für die Lippenpflege | |
|---|---|
| ad 100 | Eucerinum anhydricum |
| 10,00 | Glycerin |
| 10,00 | Titanium Dioxid |
| 1,50 | Verbindung des Beispiels 1 |
| 8,00 | Octyl Methoxycinnamat |

(fortgesetzt)

| | |
|---|---|
| 5,00 | Zink Oxid |
| 4,00 | Castoröl |
| 4,00 | Pentaerythrithyl Stearat/Caprat/Caprylat/Adipat |
| 3,00 | Glyceryl Stearat SE |
| 2,00 | Bienenwachs |
| 2,00 | Microkristallines Wachs |
| 2,00 | Quaternium-18 Bentonit |
| 1,50 | PEG-45/Dodecyl Glycol Copolymer |

Beispiel 4

| Zusammensetzung für Sunblocker mit Mikropigmenten | |
|---|---|
| ad 100 | Wasser |
| 10,00 | Octyl Methoxcinnamat |
| 6,00 | PEG-7-Hydrogenated Castor Öl |
| 6,00 | Titanium Dioxid |
| 3,00 | Verbindung des Beispiels 1 |
| 5,00 | Mineral Öl |
| 5,00 | Isoamyl p-Methoxycinnamat |
| 5,00 | Propylen Glycol |
| 3,00 | Jojoba Öl |
| 3,00 | 4-Methylbenzyliden Campher |
| 2,00 | PEG-45/Dodecyl Glycol Copolymer |
| 1,00 | Dimethicon |
| 0,50 | PEG-40-Hydrogenated Castor Öl |
| 0,50 | Tocopheryl Acetat |
| 0,50 | Phenoxyethanol |
| 0,20 | EDTA |

Beispiel 5

| Zusammensetzung für Sunblocker mit Mikropigmenten | |
|---|---|
| ad 100 | Wasser |
| 10,00 | Octyl Methoxcinnamat |
| 6,00 | PEG-7-Hydrogenated Castor Öl |
| 6,00 | Titanium Dioxid |
| 2,00 | Verbindung des Beispiels 1 |
| 5,00 | Mineral Öl |
| 5,00 | Isoamyl p-Methoxycinnamat |
| 5,00 | Propylen Glycol |
| 3,00 | Jojoba Öl |
| 3,00 | 4-Methylbenzyliden Campher |
| 2,00 | PEG-45/Dodecyl Glycol Copolymer |
| 1,00 | Dimethicon |
| 0,50 | PEG-40-Hydrogenated Castor Öl |
| 0,50 | Tocopheryl Acetat |
| 0,50 | Phenoxyethanol |

(fortgesetzt)

| | |
|---|---|
| 0,20 | EDTA |

Beispiel 6

| Fettfreies Gel | |
|---|---|
| ad 100 | Wasser |
| 8,00 | Octyl Methoxycinnamat |
| 7,00 | Titanium Dioxid |
| 1,50 | Verbindung des Beispiels 1 |
| 5,00 | Glycerin |
| 5,00 | PEG-25 PABA |
| 1,00 | 4-Methylbenzyliden Campher |
| 0,40 | Acrylate $C_{10}$-$C_{30}$ Alkyl Acrylat Crosspolymer |
| 0,30 | Imidazolidinyl Urea |
| 0,25 | Hydroxyethyl Cellulose |
| 0,25 | Sodium Methylparaben |
| 0,20 | Disodium EDTA |
| 0,15 | Fragrance |
| 0,15 | Sodium Propylparaben |
| 0,10 | Sodium Hydroxid |

Beispiel 7

| Fettfreies Gel | |
|---|---|
| ad 100 | Wasser |
| 8,00 | Octyl Methoxycinnamat |
| 7,00 | Titanium Dioxid |
| 2,00 | Verbindung des Beispiels 1 |
| 5,00 | Glycerin |
| 5,00 | PEG-25 PABA |
| 1,00 | 4-Methylbenzyliden Campher |
| 0,40 | Acrylate $C_{10}$-$C_{30}$ Alkyl Acrylat Crosspolymer |
| 0,30 | Imidazolidinyl Urea |
| 0,25 | Hydroxyethyl Cellulose |
| 0,25 | Sodium Methylparaben |
| 0,20 | Disodium EDTA |
| 0,15 | Fragrance |
| 0,15 | Sodium Propylparaben |
| 0,10 | Sodium Hydroxid |

Beispiel 8

| Sonnencreme | |
|---|---|
| ad 100 | Wasser |
| 8,00 | Octyl Methoxycinnamat |

(fortgesetzt)

| | |
|---|---|
| 8,00 | Titanium Dioxid |
| 6,00 | PEG-7-Hydrogenated Castor Öl |
| 1,00 | Verbindung des Beispiels 1 |
| 6,00 | Mineral Öl |
| 5,00 | Zink Oxid |
| 5,00 | Isopropyl Palmitat |
| 5,00 | Imidazolidinyl Urea |
| 3,00 | Jojoba Öl |
| 2,00 | PEG-45/Dodecyl Glycol Copolymer |
| 1,00 | 4-Methylbenzyliden Campher |
| 0,60 | Magnesium Stearat |
| 0,50 | Tocopheryl Acetat |
| 0,25 | Methylparaben |
| 0,20 | Disodium EDTA |
| 0,15 | Propylparaben |

Beispiel 9

Sonnencreme

| | |
|---|---|
| ad 100 | Wasser |
| 8,00 | Octyl Methoxycinnamat |
| 8,00 | Titanium Dioxid |
| 6,00 | PEG-7-Hydrogenated Castor Öl |
| 1,00 | Verbindung der Formel 1 |
| 6,00 | Mineral Öl |
| 5,00 | Zink Oxid |
| 5,00 | Isopropyl Palmitat |
| 5,00 | Imidazolidinyl Urea |
| 3,00 | Jojoba Öl |
| 2,00 | PEG-45/Dodecyl Glycol Copolymer |
| 1,00 | 4-Methylbenzyliden Campher |
| 0,60 | Magnesium Stearat |
| 0,50 | Tocopheryl Acetat |
| 0,25 | Methylparaben |
| 0,20 | Disodium EDTA |
| 0,15 | Propylparaben |

Beispiel 10

Sonnencreme wasserfest

| | |
|---|---|
| ad 100 | Wasser |
| 8,00 | Octyl Methoxycinnamat |
| 5,00 | PEG-7-Hydrogenated Castor Öl |
| 5,00 | Propylene Glycol |
| 4,00 | Isopropyl Palmitat |
| 4,00 | Caprylic/Capric Triglycerid |
| 1,50 | Verbindung der Formel 1 |

(fortgesetzt)

| | |
|---|---|
| 4,00 | Glycerin |
| 3,00 | Jojoba Öl |
| 2,00 | 4-Methylbenzyliden Campher |
| 2,00 | Titanium Dioxid |
| 1,50 | PEG-45/Dodecyl Glycol Copolymer |
| 1,50 | Dimethicon |
| 0,70 | Magnesium Sulfat |
| 0,50 | Magnesium Stearat |
| 0,15 | Fragrance |

Beispiel 11

Sonnencreme wasserfest

| | |
|---|---|
| ad 100 | Wasser |
| 8,00 | Octyl Methoxycinnamat |
| 5,00 | PEG-7-Hydrogenated Castor Öl |
| 5,00 | Propylene Glycol |
| 4,00 | Isopropyl Palmitat |
| 4,00 | Caprylic/Capric Triglycerid |
| 1,00 | Verbindung der Formel 1 |
| 4,00 | Glycerin |
| 3,00 | Jojoba Öl |
| 2,00 | 4-Methylbenzyliden Campher |
| 2,00 | Titanium Dioxid |
| 1,50 | PEG-45/Dodecyl Glycol Copolymer |
| 1,50 | Dimethicon |
| 0,70 | Magnesium Sulfat |
| 0,50 | Magnesium Stearat |
| 0,15 | Fragrance |

Beispiel 12

Sonnenmilch

| | |
|---|---|
| ad 100 | Wasser |
| 10,00 | Mineral Öl |
| 6,00 | PEG-7-Hydrogenated Castor Öl |
| 5,00 | Isopropyl Palmitat |
| 3,50 | Octyl Methoxycinnamat |
| 0,50 | Verbindung des Beispiels 1 |
| 3,00 | Caprylic/Capric Triglycerid |
| 3,00 | Jojoba Öl |
| 2,00 | PEG-45/Dodecyl Glycol Copolymer |
| 0,70 | Magnesium Sulfat |
| 0,60 | Magnesium Stearat |
| 0,50 | Tocopheryl Acetat |
| 0,30 | Glycerin |
| 0,25 | Methylparaben |

(fortgesetzt)

| | |
|---|---|
| 0,15 | Propylparaben |
| 0,05 | Tocopherol |

Beispiel 13

Sonnenmilch

| | |
|---|---|
| ad 100 | Wasser |
| 10,00 | Mineral Öl |
| 6,00 | PEG-7-Hydrogenated Castor Öl |
| 5,00 | Isopropyl Palmitat |
| 3,50 | Octyl Methoxycinnamat |
| 0,50 | Verbindung des Beispiels 1 |
| 3,00 | Caprylic/Capric Triglycerid |
| 3,00 | Jojoba Öl |
| 2,00 | PEG-45/Dodecyl Glycol Copolymer |
| 0,70 | Magnesium Sulfat |
| 0,60 | Magnesium Stearat |
| 0,50 | Tocopheryl Acetat |
| 0,30 | Glycerin |
| 0,25 | Methylparaben |
| 0,15 | Propylparaben |
| 0,05 | Tocopherol |

Beispiel 14

Sonnencreme wasserfest

| | |
|---|---|
| ad 100 | Wasser |
| 8,00 | Octyl Methoxycinnamat |
| 5,00 | PEG-7-Hydrogenated Castor Öl |
| 5,00 | Propylene Glycol |
| 4,00 | Isopropyl Palmitat |
| 4,00 | Caprylic/Capric Triglycerid |
| 2,00 | Verbindung des Beispiels 1 |
| 4,00 | Glycerin |
| 3,00 | Jojoba Öl |
| 2,00 | 4-Methylbenzyliden Campher |
| 2,00 | Titanium Dioxid |
| 1,50 | PEG-45/Dodecyl Glycol Copolymer |
| 1,50 | Dimethicon |
| 0,70 | Magnesium Sulfat |
| 0,50 | Magnesium Stearat |
| 0,15 | Fragrance |

Beispiel 15

Sonnenmilch

| | |
|---|---|
| ad 100 | Wasser |
| 10,00 | Mineral Öl |
| 6,00 | PEG-7-Hydrogenated Castor Öl |
| 5,00 | Isopropyl Palmitat |
| 3,50 | Octyl Methoxycinnamat |
| 2,00 | Verbindung des Beispiels 1 |
| 3,00 | Caprylic/Capric Triglycerid |
| 3,00 | Jojoba Öl |
| 2,00 | PEG-45/Dodecyl Glycol Copolymer |
| 0,70 | Magnesium Sulfat |
| 0,60 | Magnesium Stearat |
| 0,50 | Tocopheryl Acetat |
| 0,30 | Glycerin |
| 0,25 | Methylparaben |
| 0,15 | Propylparaben |
| 0,05 | Tocopherol |

**Patentansprüche**

1. Verwendung von Enaminotriazinen der Formel I

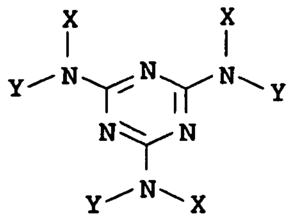

(I),

in der die Reste Y unabhängig voneinander Wasserstoff, einen gegebenenfalls substituierten aliphatischen, cycloaliphatischen, araliphatischen oder aromatischen Rest mit jeweils bis zu 18 C-Atomen bedeuten, die Reste X gleich oder verschieden sind, wobei mindestens einer der Reste X den Ethenylrest der Formel II

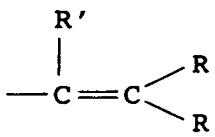

(II)

bedeutet und X ferner die Bedeutungen von Y haben kann und wobei in der Formel II die Reste Reste R gleich oder verschieden sind und Cyan, gegebenenfalls verestertes Carboxy, substituiertes Carbonyl, gegebenenfalls substituiertes Aminocarbonyl, gegebenenfalls verestertes Sulfo, substituiertes Sulfonyl oder gegebenenfalls verestertes Phosphono und wobei zwei benachbarte Reste R gegebenenfalls einen Ring bilden können
und die Reste R' gleich oder verschieden sind und Wasserstoff, einen gegebenenfalls substituierten aliphatischen, cycloaliphatischen oder aromatischen Rest mit jeweils bis 18 C-Atomen bedeuten, als UV-Filter zur Herstellung von kosmetischen oder pharmazeutischen Zubereitungen zum Schutz der menschlichen Haut oder menschlichen Haare gegen Sonnenstrahlen, allein oder zusammen mit an sich für kosmetische Zubereitungen bekannten, im UV-Bereich absorbierenden Verbindungen.

2. Verwendung von Verbindungen der Formel I gemäß Anspruch 1, **dadurch gekennzeichnet, daß** alle Reste Y Wasserstoff und alle Reste X den Ethenylrest der Formel II bedeuten.

3. Verwendung von Verbindungen gemäß Anspruch 1 der Formel I, in der Y Wasserstoff und einer oder mehrere der Reste X einen Ethenylrest der Formel IIa

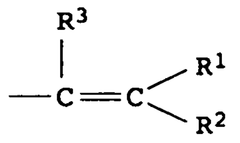

(IIa)

bedeuten, in der die Reste $R^1$ und $R^2$ gleich oder verschieden sind und $-COOR^4$, $-COR^4$, $-CON(R^4)_2$, -CN, $-SO_2R^4$, $-SO_2OR^4$ oder $-PO(OR^4)_2$ bedeuten, wobei die Reste $R^4$ unabhängig voneinander Wasserstoff einen aliphatischen, cycloaliphatischen, araliphatischen oder aromatischen Rest, die jeweils gegebenenfalls substituiert sein können, bedeuten und wobei benachbarte Reste $R^1$ und $R^2$ gegebenenfalls einen Ring bilden können und $R^3$ für Wasserstoff oder einen aliphatischen, cycloaliphatischen, araliphatischen oder aromatischen Rest, die jeweils substituiert sein können, steht.

4. Verwendung von Verbindungen der Formel I gemäß Anspruch 3, **dadurch gekennzeichnet, daß** $R^3$ Wasserstoff, $R^1$ und $R^2$ gleich oder verschieden sind und $-COOR^5$, $-COR^5$ oder -CN bedeuten, wobei $R^5$ für einen aliphatischen oder gegebenenfalls substituierten aromatischen Rest mit bis zu 18 C-Atomen steht.

5. Lichtschutzmittel enthaltende kosmetische oder pharmazeutische Zubereitungen in Form von Ölen, Öl-in-Wasser- und Wasser-in-Öl-Emulsionen, Cremes, Pasten, Lippenschutzstiftmassen oder fettfreien Gelen zum Schutz der menschlichen Haut oder menschlichen Haare gegen Sonnenstrahlen, allein oder zusammen mit an sich für kosmetische Zubereitungen bekannten, im UV-Bereich absorbierenden Verbindungen, die als wesentliches UV-Filter wirksame Mengen von Enaminotriazinen der Formel I

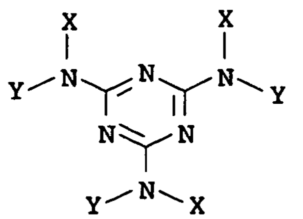

(I),

enthalten, in der die Reste Y unabhängig voneinander Wasserstoff, einen gegebenenfalls substituierten aliphatischen, cycloaliphatischen, araliphatischen oder aromatischen Rest mit jeweils bis zu 18 C-Atomen bedeuten, die Reste X gleich oder verschieden sind, wobei mindestens einer der Reste X den Ethenylrest der Formel II

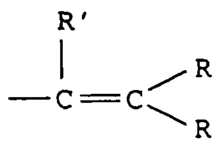

(II)

bedeutet und X ferner die Bedeutungen von Y haben kann und wobei in der Formel II die Reste Reste R gleich oder verschieden sind und Cyan, gegebenenfalls verestertes Carboxy, substituiertes Carbonyl, gegebenenfalls substituiertes Aminocarbonyl, gegebenenfalls verestertes Sulfo, substituiertes Sulfonyl oder gegebenenfalls verestertes Phosphono bedeuten und wobei zwei benachbarte Reste R gegebenenfalls einen Ring bilden können, und die Reste R' gleich oder verschieden sind und Wasserstoff, einen gegebenenfalls substituierten aliphatischen, cycloaliphatischen oder aromatischen Rest mit jeweils bis 18 C-Atomen bedeuten.

6. Lichtschutzmittel enthaltende kosmetische oder pharmazeutische Zubereitungen gemäß Anspruch 5, **dadurch gekennzeichnet, daß** sie Enaminotriazine der Formel I enthalten, in der alle Reste Y Wasserstoff und alle Reste X den Ethenylrest der Formel II bedeuten.

7. Lichtschutzmittel enthaltende kosmetische oder pharmazeutische Zubereitungen gemäß Anspruch 5, **dadurch gekennzeichnet, daß** sie Enaminotriazine der Formel I enthalten, in der alle Reste Y Wasserstoff und einer oder mehrerer Reste X den Ethenylrest der Formel IIa

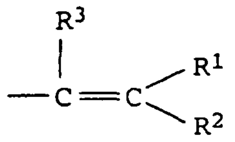

(IIa)

bedeuten, in der die Reste $R^1$ und $R^2$ gleich oder verschieden sind und $-COOR^4$, $-COR^4$, $-CON(R^4)_2$, -CN, $-SO_2R^4$, $-SO_2OR^4$ oder $-PO(OR^4)_2$ bedeuten, wobei die Reste $R^4$ unabhängig voneinander Wasserstoff einen aliphatischen, cycloaliphatischen, araliphatischen oder aromatischen Rest, die jeweils gegebenenfalls substituiert sein können, bedeuten und wobei benachbarte Reste $R^1$ und $R^2$ gegebenenfalls einen Ring bilden können und $R^3$ für Wasserstoff oder einen aliphatischen, cycloaliphatischen, araliphatischen oder aromatischen Rest, die jeweils substituiert sein können, steht.

8. Lichtschutzmittel enthaltende kosmetische oder pharmazeutische Zubereitungen gemäß Anspruch 7, **dadurch gekennzeichnet, daß** $R^3$ Wasserstoff, $R^1$ und $R^2$ gleich oder verschieden sind und $-COOR^5$, $-COR^5$ oder -CN bedeuten, wobei $R^5$ für einen aliphatischen oder gegebenenfalls substituierten aromatischen Rest mit bis zu 18 C-Atomen steht.

9. Tris-(ethenylamino)-triazine der Formel III

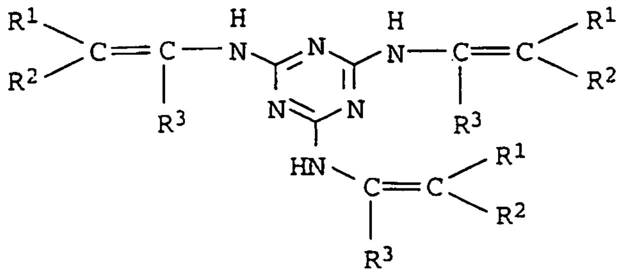

(III),

in der die Reste $R^1$ und $R^2$ gleich oder verschieden sind und $-COOR^4$, $-COR^4$, $-CON(R^4)_2$, -CN, $-SO_2R^4$, $-SO_2OR^4$ oder $-PO(OR^4)_2$ bedeuten, wobei die Reste $R^4$ unabhängig voneinander Wasserstoff einen aliphatischen, cycloaliphatischen, araliphatischen oder aromatischen Rest, die jeweils gegebenenfalls substituiert sein können, bedeuten und wobei zwei benachbarte Reste R gegebenenfalls einen Ring bilden können und $R^3$ für Wasserstoff oder einen aliphatischen, cycloaliphatischen, araliphatischen oder aromatischen Rest, die jeweils substituiert sein können, steht.

## Claims

1. The use of enaminotriazines of the formula I

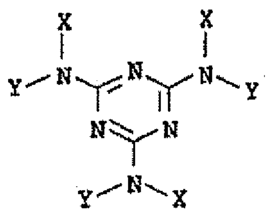

(I),

in which the radicals Y, independently of one another, are hydrogen, an optionally substituted aliphatic, cycloaliphatic,

araliphatic or aromatic radical having in each case up to 18 carbon atoms, the radicals X are identical or different, where at least one of the radicals X is the ethenyl radical of the formula II

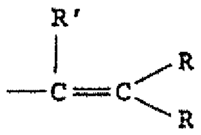

(II)

and X may also have the meanings of Y, and where in the formula II the radicals R are identical or different and are cyano, optionally esterified carboxyl, substituted carbonyl, optionally substituted aminocarbonyl, optionally esterified sulfo, substituted sulfonyl or optionally esterified phosphono, and where two adjacent radicals R may optionally form a ring and the radicals R' are identical or different and are hydrogen, an optionally substituted aliphatic, cycloaliphatic or aromatic radical having in each case up to 18 carbon atoms, as UV filters in the manufacture of cosmetic or pharmaceutical preparations for protecting human skin or human hair against solar rays, alone or together with compounds which absorb in the UV region and which are known per se for cosmetic preparations.

**2.** The use of compounds of the formula I according to claim 1, wherein all radicals Y are hydrogen and all radicals X are the ethenyl radical of the formula II.

**3.** The use of compounds according to claim 1 of the formula I in which Y is hydrogen and one or more of the radicals X are an ethenyl radical of the formula IIa

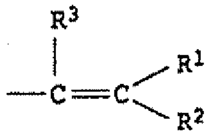

(IIa)

in which the radicals $R^1$ and $R^2$ are identical or different and are $-COOR^4$, $-COR^4$, $-CON(R^4)_2$, -CN, $-SO_2R^4$, $-SO_2OR^4$ or $-PO(OR^4)_2$, where the radicals $R^4$, independently of one another, are hydrogen, an aliphatic, cycloaliphatic, araliphatic or aromatic radical, each of which may be optionally substituted, and where adjacent radicals $R^1$ and $R^2$ may optionally form a ring
and $R^3$ is hydrogen or an aliphatic, cycloaliphatic, araliphatic or aromatic radical, each of which may be substituted.

**4.** The use of compounds of the formula I according to claim 3, wherein $R^3$ is hydrogen, $R^1$ and $R^2$ are identical or different and are $-COOR^5$, $-COR^5$ or -CN, where $R^5$ is an aliphatic or optionally substituted aromatic radical having up to 18 carbon atoms.

**5.** A cosmetic or pharmaceutical preparation in the form of oils, oil-in-water and water-in-oil emulsions, creams, pastes, lipcare stick compositions or grease-free gels comprising light protection agents for protecting human skin or human hair against solar rays, alone or together with compounds which absorb in the UV region and which are known per se for cosmetic preparations and which comprise, as essential UV filters, effective amounts of enaminotriazines of the formula I

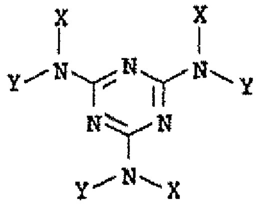

(I),

in which the radicals Y, independently of one another, are hydrogen, an optionally substituted aliphatic, cycloaliphatic,

araliphatic or aromatic radical having in each case up to 18 carbon atoms, the radicals X are identical or different, where at least one of the radicals X is the ethenyl radical of the formula II

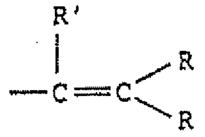 (II)

and X may also have the meanings of Y, and where in the formula II the radicals R are identical or different and are cyano, optionally esterified carboxyl, substituted carbonyl, optionally substituted aminocarbonyl, optionally esterified sulfo, substituted sulfonyl or optionally esterified phosphono, and where two adjacent radicals R may optionally form a ring, and the radicals R' are identical or different and are hydrogen, an optionally substituted aliphatic, cycloaliphatic or aromatic radical having in each case up to 18 carbon atoms.

**6.** The cosmetic or pharmaceutical preparation comprising light protection agents according to claim 5, which comprises enaminotriazines of the formula I in which all of the radicals Y are hydrogen and all of the radicals X are the ethenyl radical of the formula II.

**7.** The cosmetic or pharmaceutical preparation comprising light protection agents according to claim 5, which comprises enaminotriazines of the formula I in which all of the radicals Y are hydrogen and one or more radicals X are the ethenyl radical of the formula IIa

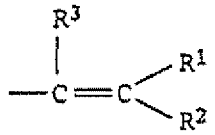 (IIa)

in which the radicals $R^1$ and $R^2$ are identical or different and are $-COOR^4$, $-COR^4$, $-CON(R^4)_2$, -CN, $-SO_2R^4$, $-SO_2OR^4$ or $-PO(OR^4)_2$, where the radicals $R^4$, independently of one another, are hydrogen, an aliphatic, cycloaliphatic, araliphatic or aromatic radical, each of which may be optionally substituted, and where adjacent radicals $R^1$ and $R^2$ may optionally form a ring and $R^3$ is hydrogen or an aliphatic, cycloaliphatic, araliphatic or aromatic radical, each of which may be substituted.

**8.** The cosmetic or pharmaceutical preparation comprising light protection agents according to claim 7, wherein $R^3$ is hydrogen, $R^1$ and $R^2$ are identical or different and are $-COOR^5$, $-COR^5$ or -CN, where $R^5$ is an aliphatic or optionally substituted aromatic radical having up to 18 carbon atoms.

**9.** A tris(ethenylamino)triazine of the formula III

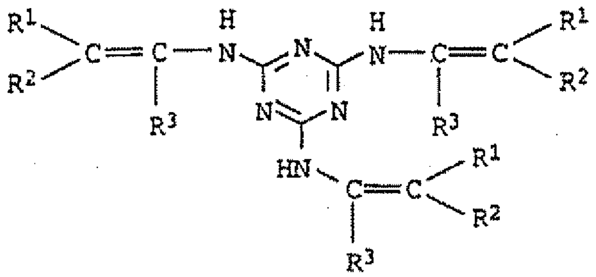 (III),

in which the radicals $R^1$ and $R^2$ are identical or different and are $-COOR^4$, $-COR^4$, $-CON(R^4)_2$, -CN, $-SO_2R^4$, $SO_2OR^4$ or $-PO(OR^4)_2$, where the radicals $R^4$, independently of one another, are hydrogen, an aliphatic, cycloaliphatic, araliphatic or aromatic radical, each of which may be optionally substituted, and where two adjacent radicals R may

optionally form a ring and $R^3$ is hydrogen or an aliphatic, cycloaliphatic, araliphatic or aromatic radical, each of which may be substituted.

## Revendications

1. Utilisation d'énaminotriazines de formule I :

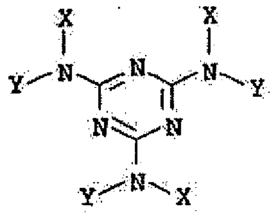

(I),

dans laquelle les radicaux Y représentent, indépendamment les uns des autres, de l'hydrogène, des radicaux aliphatique, cycloaliphatique, araliphatique ou aromatique éventuellement substitués avec, respectivement, jusqu'à 18 atomes de carbone, les radicaux X sont identiques ou différents, au moins l'un des radicaux X représentant le radical éthényle de formule II :

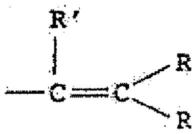

(II)

et X pouvant en outre avoir les significations de Y, et dans 1a formule II, les radicaux R étant identiques ou différents et représentant un groupe cyano, carboxy éventuellement estérifié, carbonyle substitué, aminocarbonyle éventuellement substitué, sulfo éventuellement estérifié, sulfonyle substitué ou phosphono éventuellement estérifié et deux radicaux voisins R pouvant éventuellement former un cycle,
et les radicaux R' étant identiques ou différents et représentant de l'hydrogène, un radical aliphatique, cycloaliphatique ou aromatique éventuellement substitué avec, respectivement, jusqu'à 18 atomes de carbone, sous la forme de filtres UV pour fabriquer des préparations cosmétiques ou pharmaceutiques pour protéger des rayons du soleil la peau ou le système pileux d'êtres humains, seules ou conjointement avec des composés absorbant dans la plage des UV connus en soi pour les préparations cosmétiques,

2. Utilisation de composés de formule I selon la revendication 1, **caractérisée en ce que** tous les radicaux Y représentent de l'hydrogène et tous les radicaux X représentent le radical éthényle de formule II.

3. Utilisation de composés de formule I selon la revendication 1, dans laquelle Y représente de l'hydrogène et un ou plusieurs des radicaux X représente(nt) un radical éthényle de formule IIa :

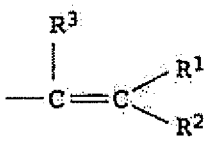

(IIa)

dans laquelle les radicaux $R^1$ et $R^2$ sont identiques ou différents et représentent un groupe $-COOR^4$, $-COR^4$, $-CON(R^4)_2$, -CN, $-SO_2R^4$, $-SO_2OR^4$ ou $-PO(OR^4)_2$, les radicaux $R^4$ représentent, indépendamment les uns des autres, de l'hydrogène, des radicaux aliphatique, cycloaliphatique, araliphatique ou aromatique, qui peuvent respectivement être éventuellement substitués, et les radicaux voisins $R^1$ et $R^2$ pouvant éventuellement former un cycle,
et $R^3$ représentant de l'hydrogène ou des radicaux aliphatique, cycloaliphatique, araliphatique ou aromatique, qui peuvent respectivement être substitués.

4. Utilisation de composés de formule I selon la revendication 3, **caractérisée en ce que** $R^3$ représente de l'hydrogène,

$R^1$ et $R^2$ sont identiques ou différents et représentent un groupe $-COOR^5$, $-COR^5$ ou -CN, $R^5$ représentant un radical aliphatique ou aromatique éventuellement substitué avec jusqu'à 18 atomes de carbone.

**5.** Préparations cosmétiques ou pharmaceutiques contenant des agents de protection contre la lumière sous la forme d'huiles, d'émulsions huile dans l'eau et eau dans l'huile, de crèmes, de pâtes, de masses pour bâton de rouge à lèvres ou de gels sans graisse pour protéger des rayons du soleil la peau ou le système pileux d'êtres humains, seules ou conjointement avec des composés absorbant dans la plage des UV connus en soi pour des préparations cosmétiques qui contiennent comme filtres à UV essentiels des quantités efficaces d'énaminotriazines de formule I :

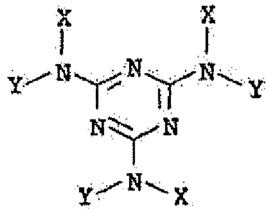

(I),

dans laquelle les radicaux Y représentent, indépendamment les uns des autres, de l'hydrogène, des radicaux aliphatique, cycloaliphatique, araliphatique ou aromatique éventuellement substitués avec, respectivement, jusqu'à 18 atomes de carbone, les radicaux X sont identiques ou différents et au moins l'un des radicaux X représentant le radical éthényle de formule II :

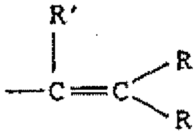

(II)

et X peut en outre avoir les significations de Y, dans la formule II, les radicaux R étant identiques ou différents et représentant un groupe cyano, carboxy éventuellement estérifié, carbonyle substitué, aminocarbonyle éventuellement substitué, sulfo éventuellement estérifié, sulfonyle substitué ou phosphono éventuellement substitué et deux radicaux voisins R pouvant éventuellement former un cycle et les radicaux R' étant identiques ou différents et représentant de l'hydrogène, des radicaux aliphatique, cycloaliphatique ou aromatique éventuellement substitués avec, respectivement, jusqu'à 18 atomes de carbone.

**6.** Préparations cosmétiques ou pharmaceutiques contenant des agents de protection contre la lumière selon la revendication 5, **caractérisées en ce qu'**elles contiennent des énaminotriazines de formule I, dans laquelle tous les radicaux Y représentent de l'hydrogène et tous les radicaux X représentent le radical éthényle de formule II.

**7.** Préparations cosmétiques ou pharmaceutiques contenant des agents de protection contre la lumière selon la revendication 5, **caractérisées en ce qu'**elles contiennent des énaminotriazines de formule I, dans laquelle tous les radicaux Y représentent de l'hydrogène et un ou plusieurs radicaux X représente(nt) le radical éthényle de formule IIa :

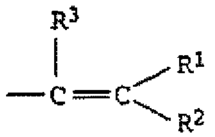

(IIa)

dans laquelle les radicaux $R^1$ et $R^2$ sont identiques ou différents et représentent un groupe $-COOR^4$, $-COR^4$, $-CON(R^4)_2$, -CN, $-SO_2R^4$, $-SO_2OR^4$ ou $-PO(OR^4)_2$, les radicaux $R_4$ représentant, indépendamment les unes des autres, de l'hydrogène, des radicaux aliphatique, cycloaliphatique, araliphatique ou aromatique qui peuvent respectivement éventuellement être substitués et des radicaux voisins $R^1$ et pouvant éventuellement former un cycle et $R^3$ représentant de l'hydrogène, des radicaux aliphatique, cycloaliphatique, araliphatique ou aromatique qui peuvent respectivement être substitués.

8. Préparations cosmétiques ou pharmaceutiques contenant des agents de protection contre la lumière selon la revendication 7, **caractérisées en ce que** $R^3$ représente de l'hydrogène, $R^1$ et $R^2$ sont identiques ou différents et représentent un groupe $-COOR^5$, $-COR^5$ ou -CN, $R^5$ représentant un radical aliphatique ou aromatique éventuellement substitué avec jusqu'à 18 atomes de carbone.

9. Tris-(éthénylamino)triazines de formule III :

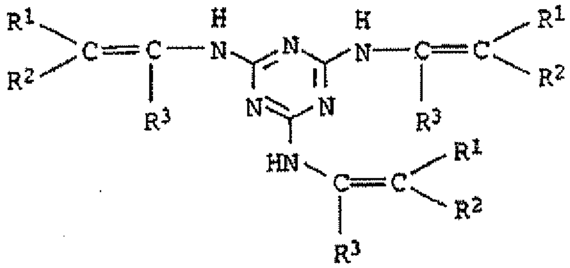

(III),

dans laquelle les radicaux $R^1$ et $R^2$ sont identiques ou différents et représentent un groupe $-COOR^4$, $-COR^4$, $-CON(R^4)_2$, -CN, $-SO_2R^4$, $-SO_2OR^4$ ou $-PO(OR^4)_2$, les radicaux $R_4$ représentant, indépendamment les uns des autres, de l'hydrogène, des radicaux aliphatique, cycloaliphatique, araliphatique ou aromatique qui peuvent respectivement être éventuellement substitués et deux radicaux voisins R pouvant éventuellement former un cycle et $R^3$ représentant de l'hydrogène ou des radicaux aliphatique, cycloaliphatique, araliphatique ou aromatique qui peuvent respectivement être substitués.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9902495 A
- WO 9847893 A
- EP 895776 A
- EP 852137 A
- EP 0852137 A


### In der Beschreibung aufgeführte Nicht-Patentliteratur


- **HASHIDA, Y ; IMAI, A. ; SEKIGUCHI, S.** *J. Heterocyclic Chem.,* 1989, vol. 26, 901-5
- **VON KREUTZBERGER, A ; GRUNDMANN, C.** *J. Org. Chem.,* 1961, vol. 4, 1121-1126
- Kosmetische Färbemittel. Farbstoffkoimmission der Deutschen Forschungsgemeinschaft. Verlag Chemie, 1984